# EUROPEAN PATENT SPECIFICATION

(11) **EP 3 851 114 B1**
(45) Date of publication and mention of the grant of the patent: **09.10.2024**
(21) Application number: 21155406.8
(22) Date of filing: 16.11.2016
(51) Int. Cl.: A61K 31/19, A61K 35/20, A61P 21/06, A61K 31/191, A61K 31/59, A61K 33/10, A61K 33/42, A61K 38/17, A61P 21/00

(54) **COMPOSITION COMPRISING MICELLAR WHEY PROTEIN, VITAMIN D AND CALCIUM FOR USE IN DECREASING FAT DEPOSITION IN SKELETAL MUSCLE IN AN ELDERLY INDIVIDUAL**
ZUSAMMENSETZUNG BEINHALTEND MIZELLARES MOLKENPROTEIN, VITAMIN D UND KALZIUM ZUR VERWENDUNG DER VERRINGERUNG VON FETTEINLAGERUNG IN DER SKELETTMUSKULATUR IN EINEM ÄLTEREN INDIVIDUUM
COMPOSITION COMPRENANT DES PROTÉINES LACTOSÉRIQUES MICELLAIRES, DE LA VITAMINE D ET DU CALCIUM POUR L'UTILISATION DANS LA RÉDUCTION DU DÉPÔT DE GRAS DANS LES MUSCLES SQUELETTAUX CHEZ UN INDIVIDU ÂGÉ

(30) Priority: 20.11.2015 US 201562258089 P
(43) Date of publication of application: 21.07.2021
(62) Divisional of application: 16797551.5
(73) Proprietor: Société des Produits Nestlé S.A., 1800 Vevey (CH)
(72) Inventor: BREUILLE, Denis, 1010 Lausanne (CH); OFFORD CAVIN, Elizabeth, 1820 Montreux (CH); ROESSLE, Claudia, 1110 Morges (CH); FIELDING, Roger, Revere, Massachusetts 2151 (US)
(74) Representative: Elkington and Fife LLP

(56) References cited:
- EP-A1- 1 839 492
- EP-A1- 2 583 563
- WO-A1-2011/011252
- WO-A1-2014/200332
- JÜRGEN M. BAUER ET AL: "Effects of a Vitamin D and Leucine-Enriched Whey Protein Nutritional Supplement on Measures of Sarcopenia in Older Adults, the PROVIDE Study: A Randomized, Double-Blind, Placebo-Controlled Trial", JOURNAL OF THE AMERICAN MEDICAL DIRECTORS ASSOCIATION, vol. 16, no. 9, 1 September 2015 (2015-09-01), NL, pages 740 - 747, XP055344311, ISSN: 1525-8610, DOI: 10.1016/j.jamda.2015.05.021
- JÜRGEN M. BAUER ET AL: "Online Supplementary Material", JOURNAL OF THE AMERICAN MEDICAL DIRECTORS ASSOCIATION, 1 September 2015 (2015-09-01), pages 1 - 1, XP055344329, Retrieved from the Internet <URL:http://www.sciencedirect.com/science/MiamiMultiMediaURL/1-s2.0-S1525861015003886/1-s2.0-S1525861015003886-mmc1.docx/273309/html/S1525861015003886/e0a7b8a11ef6770e232113f7c0982d21/mmc1.docx> [retrieved on 20170209]
- A. M. VERREIJEN ET AL: "A high whey protein-, leucine-, and vitamin D-enriched supplement preserves muscle mass during intentional weight loss in obese older adults: a double-blind randomized controlled trial", THE AMERICAN JOURNAL OF CLINICAL NUTRITION, vol. 101, no. 2, 1 February 2015 (2015-02-01), US, pages 279 - 286, XP055344319, ISSN: 0002-9165, DOI: 10.3945/ajcn.114.090290
- RONDANELLI MARIANGELA ET AL: "Whey protein, amino acids, and vitamin D supplementation with physical activity increases fat-free mass and strength, functionality, and quality of life and decreases inflammation in sarcopenic elderly", AMERICAN JOURNAL OF CLINICAL NUTRITION, vol. 103, no. 3, March 2016 (2016-03-01), pages 830 - 840, XP009193448

## Description

### BACKGROUND

The present disclosure generally relates to compositions for use which comprise whey protein, as whey protein micelles, to decrease fat deposition in muscle and/or improve or maintain muscle quality as defined in the claims. More specifically, the present disclosure relates to administering a composition comprising whey protein to an elderly individual.

Aging is associated with a progressive loss in lean mass and with an increase in the percentage of fat mass, with a redistribution of fat from subcutaneous depots to central or visceral fat accumulation. The decrease in lean mass involves mainly skeletal muscle and is accompanied by significant changes in muscle histology, with a loss in fast twitch fibers and an increase in inter-muscular adipose tissue infiltration. Inter-muscular adipose tissue accumulation has been found to be associated with the metabolic syndrome in normal and overweight elderly subjects and may produce inflammatory cytokines that could affect muscular performance. Furthermore, muscular fat infiltration was independently associated to a reduction in physical performance in some studies. In a study, a clear association between both total and fat-free muscular area of the erector spinae muscle and the physical performance score was found, and the degree of fat infiltration in the same muscle was found inversely related to the physical performance. DeStefano et al., J. Nutr. Health Aging, 19(7):785-791 (2015).

Sarcopenia is defined as the age-associated loss of muscle mass and functionality (including muscle strength and gait speed). Muscle weakness is consistently reported as an independent risk factor for high mortality in older adults.

Cross-sectional analyses of men and women aged 70 to 79 showed that lower strength with older age was predominantly due to a lower muscle mass. However, age and body fat also had significant inverse associations with strength and muscle quality. Therefore, both preservation of lean muscle mass and prevention of gain in fat mass may be important in maintaining strength and muscle quality in old age. Muscle quality is often defined as the strength per unit of muscle mass and thus is not the same as muscle mass. The loss of muscle mass in sarcopenia is associated with the decline in strength in older adults, but this strength decline is much more rapid than the accompanying loss of muscle mass, suggesting a decline in muscle quality as well. Moreover, muscle mass maintenance or gains do not prevent aging-associated declines in muscle strength. Preservation of lean mass may be important to prevent strength decline in old age, but a significant amount of the age-dependent strength decline is not explained solely by the loss of muscle mass (See Goodpaster et al., J. Gerontol. A. Biol. Sci. Med. Sci. 61(10): 1059-1064 (2006) entitled "The loss of skeletal muscle strength, mass, and quality in older adults: the health, aging and body composition study").

Bauer et al., JAMDA 16 740-747 (2015) discloses the effects of a Vitamin D and leucine-enriched whey protein nutritional supplement on measures of sarcopenia in older adults. Verreijen et al., Am. J. Clin Nutr. 101:279-86 (2015) discloses that a high whey protein-, leucine-, and vitamin D-enriched supplement preserves muscle mass during intentional weight loss in obese older adults. EP2583563A1 discloses whey protein micelles for use in the treatment and/or prevention of a condition linked to a reduced concentration of plasma amino acids in a patient. A further aspect of the invention is a meal replacement comprising whey protein micelles. Rondanelli et al., is prior art under Article 54(3) EPC and discloses that whey protein, amino acids, and vitamin D supplementation with physical activity increases fat-free mass and strength, functionality, and quality of life and decreases inflammation in sarcopenic elderly. WO 2014/200332 A1 discloses the use of a composition comprising per serving between 50 - 300 kcal; between 10 g and 35g proteinaceous matter; and at least 2.5 microgram vitamin D, for the manufacture of a nutritional product with certain muscular conditions in obese or overweight adults of at least 40 years of age participating in a weight loss program. WO 2011/011252 A1 discloses nutritional compositions and methods of using the nutritional compositions with exercise to attenuate the loss of functional status.

Indeed, aging is associated with progressive changes in total and regional fat distribution that have negative health consequences. A preferential increase in abdominal fat, in particular visceral fat, combined with a decrease in lower body subcutaneous fat is an age-related change in body composition that can occur independent of changes in total adiposity, body weight or waist circumference, and represents a phenotype closely associated with increased morbidity and mortality risk. Notably, skeletal muscle in the elderly have increased fat deposition. Kuk et al., Ageing Res. Rev. 8(4):339-48 (2009).

Nevertheless, effective measures to preserve or improve muscle strength and muscle mass remain lacking.

### SUMMARY

The present inventors conducted a clinical study on the administration of a composition comprising a high amount of whey protein to the elderly population. Surprisingly, in the elderly individuals consuming the whey protein composition, their intramuscular fat decreased more with the whey protein composition than a placebo drink control. Furthermore, the thigh muscle cross-sectional area (CSA) increased more with the whey protein composition than a placebo drink control. Without wishing to be bound by theory, these data suggest that a better muscle quality is achieved in those administered a high amount of whey protein.

Accordingly, in a general embodiment, the present disclosure provides a composition for use in decreasing fat deposition in skeletal muscle in an elderly individual or reducing the loss of skeletal muscle quality in an elderly individual; comprising administering a composition comprising whey protein to the elderly individual, the composition is administered to the elderly individual in an amount that provides at least 18 g of the whey protein per day, wherein the whey protein comprises whey protein micelles, wherein the composition further comprises vitamin D and calcium; wherein the elderly individual has sarcopenic obesity; wherein "muscle quality" is defined as the metabolic quality of skeletal muscle through fat infiltration and muscle density; wherein "elderly" relates to a human with an age from birth of at least 60 years.

The whey protein comprises whey protein micelles.

In an embodiment, the composition is administered to the elderly individual in an amount that provides at least 20 g of the whey protein per day.

The composition comprises Vitamin D and is administered in an amount that provides at least 500 IU of the Vitamin D per day. At least a portion of the Vitamin D can be selected from the group consisting of Vitamin D3; 1,25 Dihydroxy Vitamin D; 25-Hydroxy Vitamin D; and mixtures thereof.

The composition comprises calcium. At least a portion of the calcium can be selected from the group consisting of calcium carbonate, calcium citrate, calcium gluconate, calcium lactate, calcium phosphate, and mixtures thereof.

In an embodiment, the elderly individual is at least 65 years of age.

In an embodiment the elderly individual is a mobility-limited or vitamin D deficient older adult.

In an embodiment, the composition is administered to the elderly individual at least twice a week for a time period of at least one month.

In an embodiment, the composition consists essentially of the whey protein, Vitamin D and calcium.

The elderly individual has sarcopenic obesity.

An advantage of one or more embodiments provided by the present disclosure is to decrease fat deposition in muscle or reduce the loss of skeletal muscle quality in an elderly individual using a nutritional composition, such as a food product or a food supplement.

Another advantage of one or more embodiments provided by the present disclosure is to reduce, prevent or treat muscle weakness in an elderly individual using a nutritional composition, such as a food product or a food supplement.

Yet another advantage of one or more embodiments provided by the present disclosure is to provide nutritional strategies to decrease fat deposition in muscle or reduce the loss of skeletal muscle quality in an elderly individual.

Additional features and advantages are described in, and will be apparent from, the following Detailed Description and the Figures.

### BRIEF DESCRIPTION OF DRAWINGS

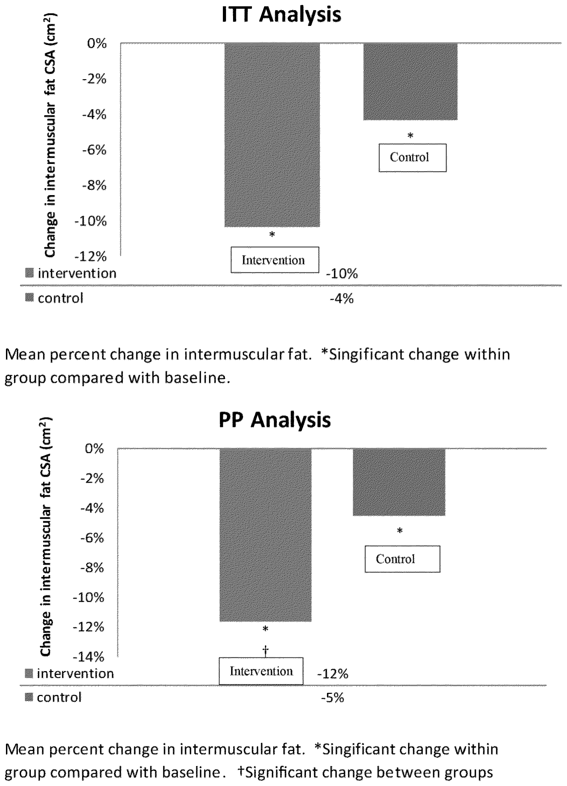

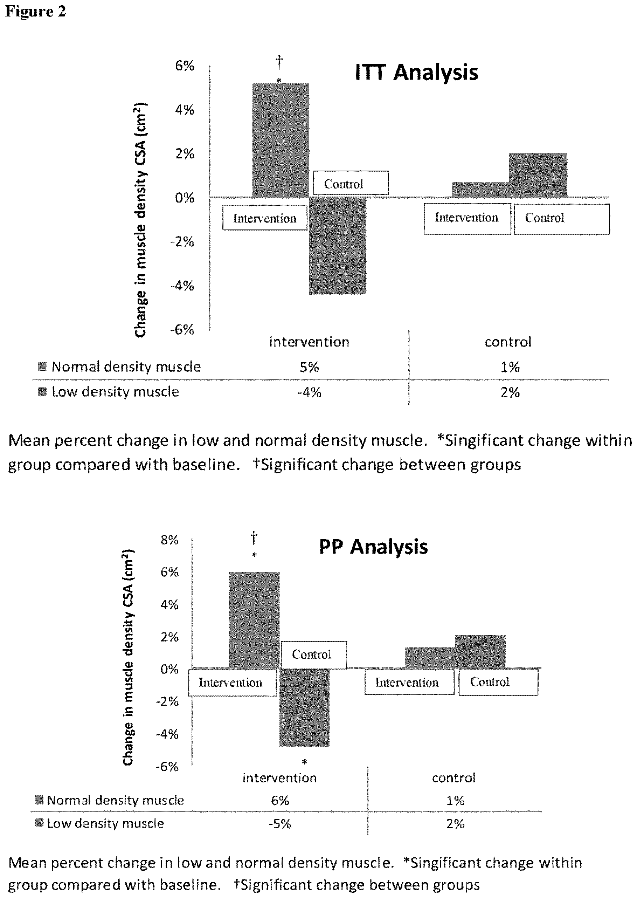

**FIGS. 1** **and** 2 show data from the clinical trial disclosed herein.

### DETAILED DESCRIPTION

### DEFINITIONS

All percentages are by weight of the total weight of the composition unless expressed otherwise. Similarly, all ratios are by weight unless expressed otherwise. "Dry weight" is the weight excluding water. When reference is made to the pH, values correspond to pH measured at 25 °C with standard equipment. As used herein, "about," "approximately" and "substantially" are understood to refer to numbers in a range of numerals, for example the range of-10% to +10% of the referenced number, preferably -5% to +5% of the referenced number, more preferably -1% to +1% of the referenced number, most preferably -0.1% to +0.1% of the referenced number.

Furthermore, all numerical ranges herein should be understood to include all integers, whole or fractions, within the range. Moreover, these numerical ranges should be construed as providing support for a claim directed to any number or subset of numbers in that range. For example, a disclosure of from 1 to 10 should be construed as supporting a range of from 1 to 8, from 3 to 7, from 1 to 9, from 3.6 to 4.6, from 3.5 to 9.9, and so forth.

As used herein and in the appended claims, the singular form of a word includes the plural, unless the context clearly dictates otherwise. Thus, the references "a," "an" and "the" are generally inclusive of the plurals of the respective terms. For example, reference to "an ingredient" or "a method" includes a plurality of such "ingredients" or "methods." The term "and/or" used in the context of "X and/or Y" should be interpreted as "X," or "Y," or "X and Y."

Similarly, the words "comprise," "comprises," and "comprising" are to be interpreted inclusively rather than exclusively. Likewise, the terms "include," "including" and "or" should all be construed to be inclusive, unless such a construction is clearly prohibited from the context. However, the embodiments provided by the present disclosure may lack any element that is not specifically disclosed herein. Thus, a disclosure of an embodiment defined using the term "comprising" is also a disclosure of embodiments "consisting essentially of" and "consisting of" the disclosed components or steps. The term "consisting essentially" means that the referenced components are at least 75% of the composition, preferably at least 85% of the composition, more preferably at least 95% of the composition, and most preferably at least 99% of the composition.

Where used herein, the term "example," particularly when followed by a listing of terms, is merely exemplary and illustrative, and should not be deemed to be exclusive or comprehensive. Any embodiment disclosed herein can be combined with any other embodiment disclosed herein unless explicitly indicated otherwise.

"Animal" includes, but is not limited to, mammals, which includes but is not limited to, rodents, aquatic mammals, domestic animals such as dogs and cats, farm animals such as sheep, pigs, cows and horses, and humans. Where "animal," "mammal" or a plural thereof is used, these terms also apply to any animal that is capable of the effect exhibited or intended to be exhibited by the context of the passage. As used herein, the term "patient" is understood to include an animal, especially a mammal, and more especially a human that is receiving or intended to receive treatment, as treatment is herein defined. While the terms "individual" and "patient" are often used herein to refer to a human, the present disclosure is not so limited. Accordingly, the terms "individual" and "patient" refer to any animal, mammal or human that can benefit from the treatment.

The term "elderly" in the context of a human means an age from birth of at least 60 years, preferably above 63 years, and more preferably above 65 years.

"Mobility-limited" older adult is defined by Short Physical Performance Battery (SPPB) ≤9

"Vitamin D insufficient" or "vitamin D deficient" older adult is defined by serum 25(OH) D 9 - 24 ng/ml.

"Overweight" is defined for a human as a BMI between 25 and 30. "Obese" is defined for a human as a BMI greater than 30. Obesity can be further classified as follows: class 1 obesity is a BMI of 30.0 to 34.9; class 2 obesity is a BMI of 35.0 to 39.9, and class 3 obesity is a BMI equal to or greater than 40.0.

The terms "treatment" and "treating" include any effect that results in the improvement of the condition or disorder, for example lessening, reducing, modulating, or eliminating the condition or disorder. The term does not necessarily imply that a subject is treated until total recovery. Non-limiting examples of "treating" or "treatment of" a condition or disorder include: (1) inhibiting the condition or disorder, i.e. arresting the development of the condition or disorder or its clinical symptoms and (2) relieving the condition or disorder, i.e. causing the temporary or permanent regression of the condition or disorder or its clinical symptoms. A treatment can be patient- or doctor-related.

The terms "prevention" or "preventing" mean causing the clinical symptoms of the referenced condition or disorder to not develop in an individual that may be exposed or predisposed to the condition or disorder but does not yet experience or display symptoms of the condition or disorder. The terms "condition" and "disorder" mean any disease, condition, symptom, or indication.

The relative terms "improved," "increased," "enhanced" refer to the effects of the composition comprising whey protein relative to a composition lacking whey protein or having less whey protein but otherwise identical. Improving or maintaining muscle quality includes reducing the loss of muscle quality.

The terms "food," "food product" and "food composition" mean a product or composition that is intended for ingestion by an individual such as a human and provides at least one nutrient to the individual. The compositions of the present disclosure, including the many embodiments described herein, can comprise, consist of, or consist essentially of the essential elements and limitations described herein, as well as any additional or optional ingredients, components, or limitations described herein or otherwise useful in a diet.

As used herein, "complete nutrition" contains sufficient types and levels of macronutrients (protein, fats and carbohydrates) and micronutrients to be sufficient to be a sole source of nutrition for the animal to which the composition is administered. Individuals can receive 100% of their nutritional requirements from such complete nutritional compositions.

"Whey protein micelles" (WPM) are defined herein as described in U.S. Patent App. Pub. No. 2009/0035437 and its counterpart EP1839492A1 and as further characterized in C. Schmitt et al., Soft Matter 6:4876-4884 (2010) where they are referred to as whey protein microgels (WPM). Particularly, the "whey protein micelles" are the micelles comprised in the whey protein micelles concentrate obtained by the process as disclosed in U.S. Patent App. Pub. No. 2009/0035437 and its counterpart EP1839492A1. Therein, the process for the production of whey protein micelles concentrate comprises the steps of: a) adjusting the pH of a whey protein aqueous solution to a value between 3.0 and 8.0; b) subjecting the aqueous solution to a temperature between 80 and 98 °C; and c) concentrating the dispersion obtained in step b). Thereby, the micelles produced have an extremely sharp size distribution, such that more than 80% of the micelles produced have a size smaller than 1 micron in diameter and preferably are between 100 nm and 900 nm in size. The "whey protein micelles" can be in liquid concentrate or in powder form. Importantly, the basic micelle structure of the whey proteins is conserved, whether in the liquid concentrate form, the powder form, or reconstituted from the powder, for example in water. The "whey protein micelles" are physically stable in dispersion, as a powder as well as during spray-drying or freeze-drying.

"Muscle weakness" is a condition where the force exerted by the muscles is less than would be expected. The U.S. Medical Research Council's grading system for muscle strength is widely used to identify muscle weakness and the severity thereof. Specifically, the examiner assesses the patient's ability to move the muscle against resistance provided by the examiner who, through experience, has developed a sense of the expected range of normal. This will vary from patient-to-patient depending upon the underlying size and conditioning of the subject; the fully trained athlete can be expected to perform differently from a small, sedentary, or deconditioned individual. The expected strength should also be adjusted for degree of atrophy in patients with wasting illnesses.

The patient's effort is graded on a scale of 0 to 5. As used herein, "muscle weakness" refers to any of grades 0-4.
Grade 5: Muscle contracts normally against full resistance.
Grade 4: Muscle strength is reduced, but muscle contraction can still move joint against resistance.
Grade 3: Muscle strength is further reduced, such that the joint can be moved only against gravity with the examiner's resistance completely removed. As an example, the elbow can be moved from full extension to full flexion starting with the arm hanging down at the side.
Grade 2: Muscle can move only if the resistance of gravity is removed. As an example, the elbow can be fully flexed only if the arm is maintained in a horizontal plane.
Grade 1: Only a trace or flicker of movement is seen or felt in the muscle, or fasciculations are observed in the muscle.
Grade 0: No movement is observed.

As used herein, "muscle quality" is the metabolic quality of skeletal muscle through fat infiltration. The common definition of "muscle quality" is strength per unit of appendicular skeletal muscle mass or per muscle volume, but "muscle quality" as used herein is more specifically directed to the metabolic quality of skeletal muscle through fat infiltration and muscle density. Non-limiting examples of ways to determine fat infiltration of muscle are computed tomography (CT) and magnetic resonance imaging (MRI).

### PREFERRED EMBODIMENTS

An aspect of the present disclosure is a composition comprising whey protein for use in decreasing fat deposition in muscle, or reducing the loss of skeletal muscle quality in an elderly individual, the use comprising administering the composition to the elderly individual; wherein the composition is administered to the elderly individual in an amount that provides at least 18 g of the whey protein per day; wherein the whey protein comprises whey protein micelles; wherein the composition further comprises vitamin D and calcium; wherein the elderly individual has the condition sarcopenic obesity; wherein "muscle quality" is defined as the metabolic quality of skeletal muscle through fat infiltration and muscle density; wherein "elderly" relates to a human with an age from birth of at least 60 years. The daily dose of the composition provides at least 18 g of whey protein, preferably at least 20 g of whey protein, and even at least 30 g of whey protein in some embodiments. In some embodiments, the decreased fat deposition in muscle and/or the improved or maintained muscle quality are achieved without modification of short term muscle functionality. In an embodiment, the elderly individual is experiencing a loss of muscle quality, and the composition reduces the loss of muscle quality.

The elderly individuals who receive the compositions of the invention is one of a specific sub-group of elderly people affected by important muscle losses, that is elderly individuals with sarcopenic obesity (as defined in the claims). Indeed, elderly individuals having sarcopenic obesity are specifically weak in term of muscle strength and exhibit strong fat infiltration.

In each of the compositions disclosed herein, is preferably administered to the individual in one or more doses per day (the aggregate thereof known as the "daily dose") for a time period that is at least one day. For example, in some embodiments the composition is administered in one single dose per day, and in other embodiments the composition is administered in multiple doses (e.g., 2-4 doses) per day that together provide a total of at least 18 g of whey protein per day (e.g., at least 20 g of whey protein per day)

The whey protein may be any whey protein, for example the whey protein can be selected from the group consisting of whey protein concentrates, whey protein isolates, whey protein micelles, whey protein hydrolysates, acid whey, sweet whey, modified sweet whey (sweet whey from which the caseino-glycomacropeptide has been removed), a fraction of whey protein, and any combination thereof. However, the whey protein comprises whey protein micelles.

The composition further comprises Vitamin D and calcium. Non-limiting examples of suitable forms of Vitamin D include Vitamin D3; 1,25 Dihydroxy Vitamin D; 25-Hydroxy Vitamin D; and mixtures thereof. Non-limiting examples of suitable forms of calcium include calcium carbonate, calcium citrate, calcium gluconate, calcium lactate, calcium phosphate, and mixtures thereof. The amount of the Vitamin D and/or the calcium can depend on a number of factors relating to the individual, such as their weight, health and how much muscle quality is being lost. However, as general non-limiting guidelines, the composition may comprise, per daily dose, 300 to 500 mg of elemental calcium and/or at least 500 IU of Vitamin D. In some embodiments, the daily dose of Vitamin D may be at least 600 UI, at least 700 UI, or at least 800 UI.

In some embodiments, the composition is administered to the individual in a single dosage form, i.e. all compounds are present in one product to be given to an individual in combination with a meal. In other embodiments, the composition is co-administered in separate dosage forms, for example the whey protein separately from one or more of the other components of the composition, and/or or a portion of the whey protein separately from another portion of the whey protein.

In some embodiments, the composition provides complete nutrition. In other embodiments, the composition is a nutritional supplement administered between meals or substantially simultaneously with a meal that is a separate composition (e.g., within fifteen minutes of the separate meal). In one particular embodiment of a nutritional supplement, the composition consists essentially of the whey protein, the Vitamin D and the calcium.

The muscle referenced in the present disclosure is a skeletal muscle. For example, the composition disclosed herein may be used to improve or maintain the muscle quality in the arms and/or the legs of the individual. The muscle may be one or more of the following: gastrocnemius, tibialis, soleus, extensor, digitorum longus (EDL), biceps femoris, semitendinosus, semimembranosus, or gluteus maximus.

The composition comprising whey protein can further comprise an additional protein source, and the additional protein source can be from animal or plant origin, for example casein, soy proteins, and/or pea proteins. Casein may be obtained from any mammal but is preferably obtained from cow milk and preferably as micellar casein. If an additional protein source is present, the composition can have any ratio of whey:other protein, for example at least 50:50, at least 60:40, or at least 70:30.

The composition can comprise one or more branched chain amino acids. For example, the composition can comprise leucine, isoleucine and/or valine. The protein source in the composition may comprise leucine in free form and/or leucine bound as peptides and/or proteins such as dairy, animal or vegetable proteins. In an embodiment, the composition comprises the leucine in an amount up to 10 wt% of the dry matter of the composition. Leucine can be present as D- or L-leucine and preferably the L-form. If the composition comprises leucine, the composition can be administered in a daily dose that provides 0.01 to 0.04 g of the leucine per kg body weight, preferably 0.02 to 0.035 g of the leucine per kg body weight. Such doses are particularly applicable to complete nutrition compositions, but one of ordinary skill will readily recognize how to adapt these doses for an oral nutritional supplement (ONS).

One or more other minerals additional to any calcium can be used in the composition. Non-limiting examples of suitable minerals include boron, chromium, copper, iodine, iron, magnesium, manganese, molybdenum, nickel, phosphorus, potassium, selenium, silicon, tin, vanadium, zinc, and combinations thereof.

One or more other vitamins additional to any Vitamin D can be used in the composition. Non-limiting examples of suitable vitamins include vitamin A, Vitamin B 1 (thiamine), Vitamin B2 (riboflavin), Vitamin B3 (niacin or niacinamide), Vitamin B5 (pantothenic acid), Vitamin B6 (pyridoxine, pyridoxal, or pyridoxamine, or pyridoxine hydrochloride), Vitamin B7 (biotin), Vitamin B9 (folic acid), and Vitamin B12 (various cobalamins; commonly cyanocobalamin in vitamin supplements), Vitamin C, Vitamin E, Vitamin K, folic acid and biotin), and combinations thereof. "Vitamin" includes such compounds obtained naturally from plant and animal foods or synthetically made, pro-vitamins, derivatives thereof, and analogs thereof.

The composition may also contain a carbohydrate and/or a source of fat. Non-limiting examples of suitable fats include canola oil, corn oil and high-oleic acid sunflower oil. Non-limiting examples of suitable carbohydrates include sucrose, lactose, glucose, fructose, corn syrup solids, maltodextrins, and mixtures thereof. Additionally or alternatively, a dietary fiber may be added. Dietary fiber passes through the small intestine undigested by enzymes and functions as a natural bulking agent and laxative. Dietary fiber may be soluble or insoluble and generally a blend of the two types is preferred. Non-limiting examples of suitable dietary fibers include soy, pea, oat, pectin, guar gum, partially hydrolyzed guar gum, gum Arabic, fructo-oligosaccharides, acidic oligosaccharides, galacto-oligosaccharides, sialyl-lactose and oligosaccharides derived from animal milks. A preferred fiber blend is a mixture of inulin with shorter chain fructo-oligosaccharides. In an embodiment, the fiber content is between 2 and 40 g/L of the composition, for example between 4 and 10 g/L.

One or more food grade emulsifiers may be incorporated into the composition, such as diacetyl tartaric acid esters of mono- and di-glycerides, lecithin, and/or mono- and di-glycerides. Suitable salts and stabilizers may be included.

The composition comprising whey protein can be administered to an individual such as a human in a therapeutically effective dose. The therapeutically effective dose can be determined by the person skilled in the art and will depend on a number of factors known to those of skill in the art, such as the severity of the condition and the weight and general state of the individual.

The composition may be administered to an individual in an amount sufficient to prevent or at least partially reduce the risk of developing muscle weakness and/or decreased muscle quality where the condition of muscle weakness or decreased muscle quality has yet not been developed in the individual. Such an amount is defined to be "a prophylactically effective dose." Again, the precise amounts depend on a number of factors relating to the individual, such as their weight, health and how much muscle quality is being lost.

The composition is preferably administered as a supplement to the diet of an individual daily or at least twice a week. In an embodiment, the composition is administered to the individual consecutively for a number of days, preferably until an increase in muscle quality relative to that before administration is achieved. For example, the composition can be administered to the individual daily for at least 30, 60 or 90 consecutive days. As another example, the composition can be administered to the individual for a longer period, such as a period of 1, 2, 3, 4, 5, 6, 7, 8, 9 or 10 years.

In a preferred embodiment, the composition is administered to the individual for at least 3 months, for example a period of 3 months to 1 year, and preferably for at least 6 months.

The above examples of administration do not require continuous daily administration with no interruptions. Instead, there may be some short breaks in the administration, such as a break of two to four days during the period of administration. The ideal duration of the administration of the composition can be determined by those of skill in the art.

In a preferred embodiment, the composition is administered to the individual orally or enterally (e.g. tube feeding). For example, the composition can be administered to the individual as a beverage, a capsule, a tablet, a powder or a suspension.

The composition can be any kind of composition that is suitable for human and/or animal consumption. For example, the composition may be selected from the group consisting of food compositions, dietary supplements, nutritional compositions, nutraceuticals, powdered nutritional products to be reconstituted in water or milk before consumption, food additives, medicaments, beverages and drinks. In an embodiment, the composition is an oral nutritional supplement (ONS), a complete nutritional formula, a pharmaceutical, a medical or a food product. In a preferred embodiment, the composition is administered to the individual as a beverage. The composition may be stored in a sachet as a powder and then suspended in a liquid such as water for use.

In some instances where oral or enteral administration is not possible or not advised, the composition may also be administered parenterally.

In an embodiment, the composition comprising whey protein further comprises a fatty acid. The fatty acid may be any fatty acid and may be one or more fatty acids, such as a combination of fatty acids. The fatty acid preferably comprises an essential fatty acid, such as the essential polyunsaturated fatty acids, namely linoleic acid (C18:2n-3) and α-linolenic acid (C18:3n-3). The fatty acid may comprise long-chain polyunsaturated fatty acids, such as eicosapentaenoic acid (C20:5n-3), arachidonic acid (C20:4n-6), docosahexaenoic acid (C22:6n-3), or any combination thereof. In a preferred embodiment, the fatty acid comprises an n-3 (omega 3) fatty acid and/or an n-6 (omega 6) fatty acid. The fatty acid preferably comprises eicosapentaenoic acid.

The fatty acid may be derived from any suitable source containing fatty acids, such as coconut oil, rapeseed oil, soya oils, corn oil, safflower oil, palm oil, sunflower oil or egg yolk. The source of the fatty acid is preferably fish oil.

As noted above, "muscle quality" as used herein is the metabolic quality of skeletal muscle through fat infiltration and thus is not evaluated using the common definition of: strength per unit of appendicular skeletal muscle mass or per muscle volume. Nevertheless, in addition to the fat disposition in the skeletal muscle which is modulated by the compositions and uses disclosed herein, in some embodiments the traditional "muscle quality" parameter can optionally be assessed as well. For example, improved metabolic quality of skeletal muscle may be evidenced by improvement in the traditional muscle quality parameter (strength per unit of appendicular skeletal muscle mass or per muscle volume). However, an improvement in the traditional muscle quality parameter does not necessarily indicate an improvement in metabolic quality of skeletal muscle.

There is no standard protocol to assess strength per unit of appendicular skeletal muscle mass or per muscle volume, but it can be analyzed using any of the approaches set forth in Barbat-Artigas et al., J. Nutr. Health Aging, 16(1):67-77 (2012), which use one or more of (i) muscle strength per unit of muscle mass, (ii) muscle power per unit of muscle mass, or (iii) muscle power alone.

In this regard, a wide range of techniques can be used to assess muscle mass, such as (i) body imaging techniques, e.g., computed tomography (CT) scan, magnetic resonance imaging (MRI), dual energy X-ray absorptiometry (DXA), and ultrasound; (ii) bio-impedance analysis (BIA), e.g., Janssen et al., J. Am. Geriatr. Soc., 50(5):889-896 (2002)) developed and validated a predictive equation for estimating skeletal muscle mass using BIA; (iii) anthropometric measures; and (iv) urinary creatinine excretion.

Muscle strength can be assessed in several muscle groups, but handgrip and knee flexors and extensors are preferred. Lower limbs are more relevant than upper limbs for gait and physical functions, but handgrip strength has been reported to be a good indicator of overall muscle strength. Two methods are mainly used to measure muscle strength: one-repetition maximum and dynamometry. One repetition maximum (1RM) is the maximum amount of weight one can lift in a single repetition for a given exercise, which can be used for determining an individual's maximum strength. Dynamometers are devices that generally allow isometric and isokinetic measurements of strength such as concentric or eccentric torque at various velocities.

Muscle power can be assessed as explosive power, i.e., the ability of muscle to perform work over a period of half a second or less, and/or short-term power i.e., the ability of muscle to perform work over a period of 12-60 seconds.

Non-limiting examples of short-term power measurements include (i) the method of Sargeant et al. (J. Appl. Physiol., 51(5):1175-1182 (1981)) in which a bicycle ergometer is modified to measure leg force and power generated at constant velocity by addition of a motor driving the pedal in a range of 23-180 revolution/min, and after an accustoming period of 15 seconds, subjects are required to make a maximum effort for 20 seconds in an attempt to speed up the motor; (ii) the Wingate anaerobic test (WAnT) which is an all-out intensity short-duration sprint cycling lasting between 10 and 40 seconds; (iii) the Non-Motorised Treadmill (NMT) Test which measures power in all-out intensity effort sprint-running; (iv) sit-to-stand tests, e.g., sit-to-stand tests in which muscle power is assessed from body mass, distance of center of gravity from sitting to standing position, and the time taken during a single chair rising, or sit-to-stand tests in which muscle power is assessed from the height of the chair, leg length, body mass, acceleration of gravity and time to perform ten chair risings.

A simple way to measure explosive muscle power is to measure the baseline 1RM, calculate several percentages of the 1RM (generally from 40% to 90%, in increments of 10%), then have the subject perform the lift at each established percentage of the 1RM as fast as possible through the full range of motion (typically starting at 40%). Depending on the muscle group assessed, the mean optima loads for maximum muscle output can range from 50 to 75% of 1RM (66, 67, 79, 80). A method more practical for an elderly subject may be that of Bassey et al. (Clin. Sci. (Lond), 82(3):321-327 (1992)) in which a leg extensor power rig consists of a seat and a footplate connected through a lever and chain to a flywheel, and the subject applies maximal force to push the footplate away and accelerate the flywheel from rest.

### EXAMPLE

The following non-limiting example presents scientific data developing and supporting the concept of administering a high amount of whey protein to an individual to decrease fat deposition in muscle in the individual; improve or maintain muscle quality in the individual; and/or treat or prevent muscle weakness in the individual.

A multicenter, double-blinded, placebo controlled randomized clinical trial assessed the augmenting effect of a nutritional supplement comprising 20 g of whey protein micelles and also Vitamin D and calcium (referenced hereafter as Intervention) on exercise training-induced changes in physical functioning, in comparison to a sham nutritional supplement (referenced hereafter as Control), in older adults with at increased risk of mobility disability. In addition to physical function measures, the trial assessed treatment effects on change in skeletal muscle (fat free) mass, lower extremity strength, fat mass and glucose control.

A total of 149 individuals were randomized and received intervention in the six-month trial. All 149 participants were retained in Intention-to-Treat (ITT) analyses discussed hereafter.

Eligibility for inclusion in the per-protocol (PP) sample was based on the following conditions: satisfaction of all enrollment (inclusion/exclusion criteria) for entry into the trial, and adherence to planned trial procedures. Per-protocol adherence to planned procedures was satisfied if subjects attended at least 60% of planned exercise sessions and utilized at least 60% of planned servings of nutritional supplement (whether Intervention or Control). Based on a six-month intervention time, the expected number of exercise sessions was 75 and nutritional supplementations was 175. A total of 120 participants were retained in PP analyses.

Regional changes in mid-thigh skeletal muscle cross sectional area were assessed by CT (Computed Tomography). Scans of the non-dominant thigh were performed at the midpoint of the femur for each subject. The length of the femur was determined from a coronal scout image as the distance between the intercondylar notch and the trocanteric notch. All scans were obtained using a Siemens Somatom Scanner (Erlangen, Germany) operating at 120 KV and 100 mA. Technical factors included a slice width of 10 mm and a scanning time of 1s. All scans were analyzed by a single investigator in a blinded manner using SliceOmatic v4.2 software (Montreal, Canada). Images were analyzed for muscle cross sectional area, and subcutaneous and intermuscular fat cross sectional area.

**Table1** provides a description of the ITT sample by trial arm. Arms are comparable in all respects, including demographics, basic anthropometries, physical functioning by short physical performance battery (SPPB), medical history, and adherence.

The resultant data demonstrates that intramuscular fat decreases more with the nutritional supplement than control (Fig 1) and that the thigh muscle cross-sectional area (CSA) increases more with the nutritional supplement than control (Fig 2). These data can be interpreted as a better muscle quality from the nutritional supplement relative to control.

Regarding proportionate change, descriptive statistics for six-month percent change - defined as 100 × (six month measurement minus baseline measurement) divided by baseline measurement - in body composition parameters is provided in **Tables** 2 and 3 for the ITT and PP samples, respectively. Other physical performance outcomes are detailed in **Table** 4 which provides summaries for the ITT sample.

**Table 1: Description of ITT sample, N=149; Mean (Standard Deviation) or N (Percent) shown.**

| | Control (N=75) | Intervention (N=74) |
|---|---|---|
| Age, years | 76.9 (4.9) | 78.1 (5.8) |
| Female, n (%) | 35 (47) | 34 (46) |
| Weight, kg | 79.8 (13.3) | 80.4 (12.6) |
| Height, cm | 167.4 (9.7) | 169.7 (9.2) |
| BMI, kg/m² | 28.4 (3.9) | 27.9 (3.3) |
| Number of Diagnoses | 2.8 (2.7) | 2.8 (2.5) |
| Number of Prescriptions | 3.9 (3.7) | 4.0 (3.8) |
| SPPB Score | 8.0 (1.1) | 7.8 (1.3) |
| MMSE Score | 27.4 (1.9) | 27.3 (1.7) |
| Serum 25(OH)D, ng/ml | 17.7 (5.9) | 19.7 (6.8) |
| *^{a}*Exercise Adherence Score | 0.72 (0.24) | 0.75 (0.22) |
| Participants Exercise Adherent, n(%) | 62 (83) | 62 (84) |
| *^{b}*Product Adherence Score | 0.86 (0.28) | 0.88 (0.22) |
| Participants Product Adherent, n(%) | 65 (87) | 65 (88) |
| *^{c}*Participants Adherent Overall, n(%) | 62 (83) | 60 (81) |
| *^{d}*Per Protocol Sample, n(%) | 60 (80) | 60 (81) |

| | | |
|---|---|---|
| *^{a}*Proportion of planned exercise visits attended *^{b}*Proportion of planned supplements taken *^{c}*Participants with both exercise and product compliance scores at lease 0.60 *^{d}*Excludes two individuals with out-of-range BMI values at screening | | |

**Table 2: Percent Change in Body Composition Parameters, ITT Sample, N=149; Means and 95% Confidence Intervals Shown.**

| | Control | Intervention | Difference |
|---|---|---|---|
| Weight, 6 months, kg | -0.78 (-1.45, -0.18) | -0.86 (-1.74, -0.02) | -0.07 (-1.08, 0.94) |
| Fat Mass, 6 Months, kg | -3.7 (-4.9, -2.5) | -2.0 (-3.5, -0.4) | 1.7 (-0.2, 3.7) |
| Lean Mass, 6 Months, kg | 0.83 (-0.04, 1.69) | -0.04 (-1.03, 0.83) | -0.87 (-2.12, 0.41) |
| Appendicular Lean Mass, 6 Months, kg | 0.4 (-0.6, 1.4) | 0.6 (-0.6, 1.7) | 0.2 (-1.3, 1.7) |
| Subcutaneous Fat, 6 Months, cm² | 0.4 (-1.6, 1.4) | 0.6 (-0.6, 1.7) | 0.2 (-1.3, 1.7) |
| Intramuscular Fat, 6 Months, Cm² | -4.4 (-8.4, -0.5) | -7.6 (-11.1, -4.2) | -3.2 (-8.2, 2.1) |
| Mid-thigh Muscle CSA, 6 Months, cm² | 1.1 (0.2, 1.9) | 2.9 (1.6, 4.3) | 1.7 (0.2, 3.4) |

**Table 3: Percent Change in Body Composition Parameters, PP Sample, N=120; Means and 95% Confidence Intervals Shown.**

| | Control | Intervention | Difference |
|---|---|---|---|
| Weight, 6 months, kg | -0.55 (-1.23, 0.12) | -0.54 (-1.35, 0.25) | -0.01 (-0.98, 1.11) |
| Fat Mass, 6 Months, kg | -3.46 (-4.89, -1.96) | -1.80 (-3.44, -0.06) | 1.67 (-0.42, 3.94) |
| Lean Mass, 6 Months, kg | 0.94 (-0.03, 1.85) | 0.29 (-0.46, 1.03) | -0.65 (-1.78, 0.72) |
| Appendicular Lean Mass, 6 Months, kg | 0.5 (-0.7, 1.6) | 0.9 (-0.1, 2.0) | 0.4 (-1.2, 2.0) |
| Subcutaneous Fat, 6 Months, cm² | -8.73 (-12.18, 4.59) | -8.66 (-12.90, -4.46) | 0.06 (-5.78, 5.41) |
| Intramuscular Fat, 6 Months, Cm² | -4.5 (-8.5, -0.4) | -8.0 (-11.7, -3.5) | -3.5 (-9.2, 2.3) |
| Mid-thigh Muscle CSA, 6 Months, cm² | 1.6 (0.7, 2.4) | 3.4 (2.1, 4.8) | 1.9 (0.2, 3.5) |

**Table 4: Summary of Physical Function Outcomes, N=149; Means and Standard**

| | | | | | | |
|---|---|---|---|---|---|---|
| Deviation Shown | | | | | | |

| | Control | | | Intervention | | |
|---|---|---|---|---|---|---|
| | BA (N=75) | 3^{MV} (N=71) | 6MV (N=68) | BA (N=74) | 3^{MV} (N=69) | 6MV (N=70) |
| SPPB Composite | 8.0 (1.1) | 10.3 (1.7) | 10.6 (1.5) | 7.8 (1.3) | 9.6 (2.1) | 9.9 (2.2) |

### Conclusion

These descriptive statistics suggest notable gains in functioning and changes in selected body composition parameters in both arms, suggesting meaningful differences attributable to the underlying exercise regime in which both arms participated. The evidence is suggestive of advantages in intramuscular fat changes and muscle density from the intervention. Thus, the addition of the nutritional supplementation according to the present invention resulted in a greater decline in intermuscular fat and improved muscle density. These results suggest such nutritional supplementation provides additional benefits in mobility-limited and vitamin D deficient older adults.

## Claims

1. A composition comprising whey protein for use in decreasing fat deposition in skeletal muscle in an elderly individual, or reducing the loss of skeletal muscle quality in an elderly individual;
wherein the composition is administered to the elderly individual in an amount that provides at least 18 g of the whey protein per day;
wherein the whey protein comprises whey protein micelles;
wherein the composition further comprises vitamin D and calcium;
wherein the elderly individual has the condition sarcopenic obesity;
wherein "muscle quality" is defined as the metabolic quality of skeletal muscle through fat infiltration and muscle density;
wherein "elderly" relates to a human with an age from birth of at least 60 years.

2. The composition for use according to claim 1, wherein the composition is administered to the elderly individual in an amount that provides at least 20 g of the whey protein per day.

3. The composition for use according to any preceding claim, wherein the composition is administered to the elderly individual in an amount that provides at least 30 g of the whey protein per day.

4. The composition for use according to any preceding claim, wherein the composition is administered in one or more doses per day, for example, one single dose per day or in multiple doses per day, such as 2 to 4 doses per days, that together provide the total of at least 18 g of whey protein per day.

5. The composition for use according to any preceding claim, wherein the whey protein is whey protein micelles.

6. The composition for use according to any preceding claim, wherein the vitamin D is selected from the group consisting of vitamin D3; 1,25-dihydroxy vitamin D; 25-hydroxy vitamin D; and mixtures thereof.

7. The composition for use according to any preceding claim, wherein the composition provides the vitamin D is at least 500 IU of Vitamin D per daily dose, at least 600 UI of Vitamin D per daily dose, at least 700 UI of Vitamin D per daily dose, or at least 800 UI of Vitamin D per daily dose.

8. The composition for use according to any preceding claim, wherein the calcium is selected from the group consisting of calcium carbonate, calcium citrate, calcium gluconate, calcium lactate, calcium phosphate, and mixtures thereof.

9. The composition for use according to any preceding claim, wherein the composition comprises 300 to 500 mg of elemental calcium per daily dose.

10. The composition for use according to any preceding claim, wherein composition comprises one or more minerals additional to calcium and/or one or more vitamins additional to vitamin D.

11. The composition for use according to any preceding claim, wherein composition comprises a carbohydrate and/or fat source; a dietary fiber; and/or one or more food grade emulsifiers.

12. The composition for use according to any preceding claim, wherein the composition is administered as a supplement to the diet of an individual daily or at least twice a week, preferably the composition is administered for at least 3 months.

13. The composition for use according to any preceding claim, wherein muscle quality is determined using computed tomography (CT) and/or magnetic resonance imaging (MRI).

## Patentansprüche

1. Zusammensetzung, umfassend Molkenprotein, zur Verwendung bei einem Verringern einer Fettablagerung in einem Skelettmuskel bei einer älteren Person oder einem Reduzieren des Verlusts von Skelettmuskelqualität bei einer älteren Person;
wobei die Zusammensetzung der älteren Person in einer Menge verabreicht wird, die mindestens 18 g des Molkenproteins pro Tag bereitstellt;
wobei das Molkenprotein Molkenproteinmizellen umfasst;
wobei die Zusammensetzung ferner Vitamin D und Calcium umfasst;
wobei die ältere Person die Krankheit sarkopenische Adipositas aufweist;
wobei "Muskelqualität" als die metabolische Qualität des Skelettmuskels durch Fetteinlagerung und Muskeldichte definiert ist;
wobei sich "älter" auf einen Menschen mit einem Alter ab Geburt von mindestens 60 Jahren bezieht.

2. Zusammensetzung zur Verwendung nach Anspruch 1, wobei die Zusammensetzung der älteren Person in einer Menge verabreicht wird, die mindestens 20 g des Molkenproteins pro Tag bereitstellt.

3. Zusammensetzung zur Verwendung nach einem der vorstehenden Ansprüche, wobei die Zusammensetzung der älteren Person in einer Menge verabreicht wird, die mindestens 30 g des Molkenproteins pro Tag bereitstellt.

4. Zusammensetzung zur Verwendung nach einem der vorstehenden Ansprüche, wobei die Zusammensetzung in einer oder mehreren Dosen pro Tag verabreicht wird, beispielsweise einer einzelnen Dosis pro Tag oder in mehrfachen Dosen pro Tag, wie 2 bis 4 Dosen pro Tag, die zusammen die Summe von mindestens 18 g Molkenprotein pro Tag bereitstellen.

5. Zusammensetzung zur Verwendung nach einem der vorstehenden Ansprüche, wobei es sich bei dem Molkenprotein um Molkenproteinmizellen handelt.

6. Zusammensetzung zur Verwendung nach einem der vorstehenden Ansprüche, wobei das Vitamin D aus der Gruppe ausgewählt ist, bestehend aus Vitamin D3; 1,25-Dihydroxyvitamin D; 25-Hydroxyvitamin D; und Mischungen davon.

7. Zusammensetzung zur Verwendung nach einem der vorstehenden Ansprüche, wobei die Zusammensetzung, die das Vitamin D bereitstellt, mindestens 500 IE Vitamin D pro Tagesdosis, mindestens 600 IE Vitamin D pro Tagesdosis, mindestens 700 IE Vitamin D pro Tagesdosis oder mindestens 800 IE Vitamin D pro Tagesdosis beträgt.

8. Zusammensetzung zur Verwendung nach einem der vorstehenden Ansprüche, wobei das Calcium aus der Gruppe ausgewählt ist, bestehend aus Calciumcarbonat, Calciumcitrat, Calciumgluconat, Calciumlactat, Calciumphosphat und Mischungen davon.

9. Zusammensetzung zur Verwendung nach einem der vorstehenden Ansprüche, wobei die Zusammensetzung 300 bis 500 mg elementares Calcium pro Tagesdosis umfasst.

10. Zusammensetzung zur Verwendung nach einem der vorstehenden Ansprüche, wobei die Zusammensetzung zusätzlich zu Calcium ein oder mehrere Mineralien und/oder zusätzlich zu Vitamin D ein oder mehrere Vitamine umfasst.

11. Zusammensetzung zur Verwendung nach einem der vorstehenden Ansprüche, wobei die Zusammensetzung ein Kohlenhydrat und/oder eine Fettquelle; einen Ballaststoff; und/oder einen oder mehrere Emulgatoren in Lebensmittelqualität umfasst.

12. Zusammensetzung zur Verwendung nach einem der vorstehenden Ansprüche, wobei die Zusammensetzung als ein Ergänzungsmittel zu der Ernährung einer Person täglich oder mindestens zweimal wöchentlich verabreicht wird, wobei vorzugsweise die Zusammensetzung für mindestens 3 Monate verabreicht wird.

13. Zusammensetzung zur Verwendung nach einem der vorstehenden Ansprüche, wobei die Muskelqualität unter Verwendung von Computertomografie (CT) und/oder Magnetresonanztomografie (MRT) bestimmt wird.

## Revendications

1. Composition comprenant de la protéine de lactosérum pour utilisation dans la diminution d'un dépôt de matière grasse dans un muscle squelettique d'une personne âgée, ou la réduction de la perte de qualité de muscle squelettique chez une personne âgée ;
dans laquelle la composition est administrée à la personne âgée en une quantité qui fournit au moins 18 g de la protéine de lactosérum par jour ;
dans laquelle la protéine de lactosérum comprend des micelles de protéines de lactosérum ;
dans laquelle la composition comprend en outre de la vitamine D et du calcium ;
dans laquelle la personne âgée a la condition d'obésité sarcopénique ;
dans laquelle la « qualité de muscle » est définie comme la qualité métabolique d'un muscle squelettique par le biais d'une infiltration de matière grasse et d'une densité musculaire ;
dans laquelle « âgé » se rapporte à un humain avec un âge à partir de la naissance d'au moins 60 ans.

2. Composition pour utilisation selon la revendication 1, dans laquelle la composition est administrée à la personne âgée en une quantité qui fournit au moins 20 g de la protéine de lactosérum par jour.

3. Composition pour utilisation selon l'une quelconque revendication précédente, dans laquelle la composition est administrée à la personne âgée en une quantité qui fournit au moins 30 g de la protéine de lactosérum par jour.

4. Composition pour utilisation selon l'une quelconque revendication précédente, dans laquelle la composition est administrée en une ou plusieurs doses par jour, par exemple, une dose unique par jour ou en de multiples doses par jour, telles que 2 à 4 doses par jours, qui fournissent ensemble le total d'au moins 18 g de protéine de lactosérum par jour.

5. Composition pour utilisation selon l'une quelconque revendication précédente, dans laquelle la protéine de lactosérum est des micelles de protéines de lactosérum.

6. Composition pour utilisation selon l'une quelconque revendication précédente, dans laquelle la vitamine D est choisie dans le groupe constitué par vitamine D3 ; 1,25-dihydroxy vitamine D ; 25-hydroxy vitamine D ; et mélanges de celles-ci.

7. Composition pour utilisation selon l'une quelconque revendication précédente, dans laquelle la composition fournit la vitamine D à raison d'au moins 500 IU de vitamine D par dose journalière, au moins 600 UI de vitamine D par dose journalière, au moins 700 UI de vitamine D par dose journalière, ou au moins 800 UI de vitamine D par dose journalière.

8. Composition pour utilisation selon l'une quelconque revendication précédente, dans laquelle le calcium est choisi dans le groupe constitué par carbonate de calcium, citrate de calcium, gluconate de calcium, lactate de calcium, phosphate de calcium, et mélanges de ceux-ci.

9. Composition pour utilisation selon l'une quelconque revendication précédente, dans laquelle la composition comprend 300 à 500 mg de calcium élémentaire par dose journalière.

10. Composition pour utilisation selon l'une quelconque revendication précédente, dans laquelle la composition comprend un ou plusieurs minéraux en supplément du calcium et/ou une ou plusieurs vitamines en supplément de la vitamine D.

11. Composition pour utilisation selon l'une quelconque revendication précédente, dans laquelle la composition comprend une source de glucides et/ou de matières grasses ; une fibre diététique ; et/ou un ou plusieurs émulsifiants de qualité alimentaire.

12. Composition pour utilisation selon l'une quelconque revendication précédente, dans laquelle la composition est administrée en tant que complément au régime alimentaire d'une personne quotidiennement ou au moins deux fois par semaine, de préférence la composition est administrée pendant au moins 3 mois.

13. Composition pour utilisation selon l'une quelconque revendication précédente, dans laquelle la qualité de muscle est déterminée à l'aide d'une tomodensitométrie (CT) et/ou d'une imagerie à résonance magnétique (IRM).
